(19) **Europäisches Patentamt / European Patent Office / Office européen des brevets**

(11) **EP 3 967 180 B1**

(12) **EUROPEAN PATENT SPECIFICATION**

(45) Date of publication and mention
of the grant of the patent:
**03.05.2023 Bulletin 2023/18**

(51) International Patent Classification (IPC):
*D04H 3/013* (2012.01)   *D04H 3/016* (2012.01)
*D04H 3/11* (2012.01)   *A45D 44/00* (2006.01)
*A61K 8/02* (2006.01)   *A61K 8/73* (2006.01)
*A61Q 19/00* (2006.01)   *B01D 39/08* (2006.01)
*B01D 39/18* (2006.01)   *A62B 23/02* (2006.01)

(21) Application number: **20802754.0**

(22) Date of filing: **08.05.2020**

(52) Cooperative Patent Classification (CPC):
**A45D 44/002; A61K 8/0212; A61K 8/027;
A61K 8/731; A61Q 19/00; B01D 39/083;
B01D 39/18; D04H 3/013; D04H 3/016; D04H 3/11;**
A62B 23/025; B01D 2239/0627; B01D 2239/0663;
B01D 2239/1233; B01D 2239/1291

(86) International application number:
**PCT/JP2020/018723**

(87) International publication number:
**WO 2020/226181 (12.11.2020 Gazette 2020/46)**

(54) **NONWOVEN FABRIC AND USE THEREOF AS A CHEMICAL IMPREGNATION SHEET**

FASERVLIESSTOFF UND VERWENDUNG DAVON ALS CHEMISCHES IMPRÄGNIERBLATT

TISSU NON-TISSÉ ET SON UTILISATION COMME FEUILLE IMPRÉGNÉE CHIMIQUEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.05.2019 JP 2019089012**

(43) Date of publication of application:
**16.03.2022 Bulletin 2022/11**

(73) Proprietor: **Asahi Kasei Kabushiki Kaisha
Tokyo 100-0006 (JP)**

(72) Inventors:
• **TANAKA, Masanobu**
**Tokyo 100-0006 (JP)**
• **UME, Shogo**
**Tokyo 100-0006 (JP)**
• **NONAKA, Shunsuke**
**Tokyo 100-0006 (JP)**
• **MACHIOKA, Kyoko**
**Tokyo 100-0006 (JP)**
• **MERA, Yuji**
**Tokyo 100-0006 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
JP-A- 2005 008 546   JP-A- 2009 297 535
JP-A- 2013 124 435   JP-A- 2015 070 968
JP-A- 2016 037 674   JP-A- 2017 150 110
JP-A- 2018 127 744   JP-A- 2018 153 632

**Description**

FIELD

The present invention relates to a regenerated cellulosic fiber nonwoven fabric, to a product that includes the nonwoven fabric, such as a chemical impregnation sheet or face mask.

BACKGROUND

With increasing attention being focused on health and beauty, chemical impregnation sheets for anti-aging are in ever rising demand. The preferred base materials used are regenerated cellulosic continuous filament nonwoven fabrics, typical of which is cupro produced by a cuprammonium rayon method, and regenerated cellulosic staple fiber nonwoven fabrics, typical of which is rayon. The reason for the wide usage of regenerated cellulosic fiber nonwoven fabrics is their high hydrophilicity compared to synthetic fiber nonwoven fabrics, and therefore higher liquid retention for chemical solutions and more satisfactory liquid holding volume.

With ever widening demand in recent years, there is an increasing desire for chemical impregnation sheets that, even with a low basis weight, have excellent handleability when wet, high adhesiveness and an excellent tactile property.

In PTL 1, a nonwoven fabric layer composed of nanofibers of a thermoplastic resin (hydrophobic fibers) is used as an attachment surface. However, the nanofibers described in PTL 1, while having satisfactory handleability when wet, have inferior hydrophilicity and low flexibility due to the use of a thermoplastic resin, while they also fail to exhibit adhesiveness on a fully satisfactory level.

PTL 2 proposes a nonwoven fabric composed of regenerated cellulosic staple fibers. The nonwoven fabric described in PTL 2 uses superfine staple fibers and has multiple layers by high-pressure stream jet tangling, whereby it exhibits improved fiber interlacing force and satisfactory handleability when wet. However, since a high-pressure stream jet is used several times during hydroentangling, a fully satisfactory level has not yet been achieved because of numerous surface irregularities, and from the viewpoint of the adhesiveness and tactile property.

PTL 3 proposes a nonwoven fabric composed of regenerated cellulosic continuous long fibers. When the nonwoven fabric described in PTL 3 is formed into a nonwoven fabric, rewetted and dried, this produces expansion and contraction of the fibers and improves the shape stability, so that the handleability is satisfactory when wet. However, expansion and contraction of the fibers results in numerous surface irregularities, and the fabric is not fully satisfactory from the viewpoint of the adhesiveness and tactile property. Thus, conventional nonwoven fabrics are still in need of improvement because they fail to simultaneously satisfy high levels for handleability when wet, adhesiveness and tactile property.

Nonwoven fabrics comprising regenerated cellulose continuous long fibers and having a specific initial tensile strength in the weft direction of the fabric are disclosed in PTL4.

[CITATION LIST]

[PATENT LITERATURE]

[PTL 1] Japanese Patent Publication No. 4816312
[PTL 2] Japanese Patent Publication No. 6385191
[PTL 3] Japanese Patent Publication No. 6267913
[PTL 4] Japanese Patent Application No. 2018/127744

SUMMARY

[TECHNICAL PROBLEM]

In light of the aforementioned problems of the prior art, the problem to be solved by the invention is to provide a nonwoven fabric that, even with low basis weight, has excellent handleability when wet, high adhesiveness and an excellent tactile property, as well as a product such as a chemical impregnation sheet employing it.

[SOLUTION TO PROBLEM]

As a result of much experimentation with the purpose of solving the problem mentioned above, the present inventors have completed this invention upon finding, unexpectedly, that the problem can be solved with a regenerated cellulosic fiber nonwoven fabric wherein the fiber size of the constituent fibers, the dry surface roughness, the dry formation index, and the initial tensile strength in the weft direction of the nonwoven fabric when wet, are within specified ranges.

Specifically, the present invention provides the following.

[1] A nonwoven fabric composed of regenerated cellulosic fibers with a fiber size of 0.5 $\mu$m to 10.0 $\mu$m, wherein the nonwoven fabric has a dry surface roughness of 100 $\mu$m or lower, a dry formation index of 250 or less, and an initial tensile strength in the weft direction of the nonwoven fabric when wet of 0.25 N/50 mm to 0.75 N/50 mm.

[2] The nonwoven fabric according to [1] above, wherein the basis weight of the nonwoven fabric is 10 g/m$^2$ to 80 g/m$^2$, and the thickness is 0.05 mm to 0.50 mm.

[3] The nonwoven fabric according to [1] or [2] above, wherein the cellulosic fibers are continuous long fibers.

[4] The nonwoven fabric according to any one of [1] to [3] above, wherein the bulk density is 0.15 g/cm$^3$ to 0.30 g/cm$^3$.

[5] Use of the nonwoven fabric according to any one of [1] to [4] above as a chemical impregnation sheet.

[6] Use according to [5] above, wherein the chemical impregnation sheet is a face mask.

[ADVANTAGEOUS EFFECTS OF INVENTION]

The regenerated cellulosic fiber nonwoven fabric of the invention is a nonwoven fabric material that, even with a low basis weight, has excellent handleability when wet, high adhesiveness and an excellent tactile property, and it can therefore be suitably used as a base material for a chemical impregnation sheet such as a face mask, point sheet or three-dimensional mask.

DESCRIPTION OF EMBODIMENTS

The invention will now be explained in detail using an embodiment.

The regenerated cellulosic fiber nonwoven fabric of the embodiment is a nonwoven fabric composed of regenerated cellulosic fibers with a fiber size of 0.5 $\mu$m to 10.0 $\mu$m, wherein the nonwoven fabric has a dry surface roughness of 100 $\mu$m or lower, a dry formation index of 250 or less, and an initial tensile strength in the weft direction when wet of 0.25 N/50 mm to 0.75 N/50 mm.

The recycled cellulosic fibers composing the nonwoven fabric are not particularly restricted and may be recycled cellulosic fibers of cuprammonium rayon, viscose rayon, Tencel (Lyocell) or polynosic. The recycled cellulosic fibers are preferably cuprammonium rayon. The fibers may be continuous long fibers or staple fibers, but continuous long fibers are preferred because they have fewer fiber ends and fluff and because they form three-dimensional tangling due to self-adhesion and high-pressure liquid flow, while causing less chemical and physical irritation on skin and having the fibers oriented in the plane, thus exhibiting more excellent adhesiveness.

The regenerated cellulosic fiber nonwoven fabric of the embodiment has a fine fiber size, low surface roughness and a low formation index, because the production method is controlled, including the spinning and hydroentangling conditions. The present inventors have found that if these properties are adjusted and combined in optimal ranges in a nonwoven fabric, the adhesiveness and tactile property are improved when the fabric is used as a chemical impregnation sheet. Specifically, chemical impregnation sheets must exhibit satisfactory adhesiveness and tactile properties, and this can be achieved by optimally controlling the fiber size, surface roughness and formation index.

If an optionally adjusted fine fiber size, low surface roughness and low formation index are exhibited, then it becomes possible to provide a nonwoven fabric with excellent handleability when wet, high adhesiveness and an excellent tactile property, even when it has a low basis weight.

The fiber size of the fibers composing the regenerated cellulosic fiber nonwoven fabric of the embodiment is 0.5 $\mu$m to 10.0 $\mu$m, preferably 1.0 $\mu$m to 10.0 $\mu$m, more preferably 1.0 $\mu$m to 9.0 $\mu$m and even more preferably 1.0 $\mu$m to 8.0 $\mu$m. If the fiber size is too small the monofilament strength will be reduced, thus increasing the ductility and impairing the handleability. If the fiber size is too large, on the other hand, the number of self-adhesion points and degree of tangling will decrease, resulting in greater ductility and impaired handleability. The surface will also tend to become sparse and the adhesiveness and tactile property will be reduced. In order to adjust the fiber size to this range it is effective to reduce the spinneret diameter, lower the spinning temperature and lower the cellulose concentration in the spinning stock solution.

The dry surface roughness of the regenerated cellulosic fiber nonwoven fabric of the embodiment is 100 $\mu$m or lower, preferably 90 $\mu$m or lower and more preferably 80 $\mu$m or lower. If the dry surface roughness is greater than

100 $\mu$m then physical irritation on the skin will increase and the tactile property will be reduced. More gaps with the skin will also form, making it less able to follow the skin and reducing adhesiveness on the skin. In order to adjust the dry surface roughness to within this range it is effective to use a fabric comprising a fine mesh as a lower layer net laminated with the spun sheet, with a mesh number of 60 or greater being especially preferred. It is also effective to increase the number of nozzles and to increase the number of water streams for hydroentangling.

The term "dry surface roughness" used herein refers to the root mean square of the heights at each of the points in the measured region, and it corresponds to the standard deviation for "height". The term "height" in this case refers to the distance from the reference surface at each point on the measurement surface.

The dry formation index of the regenerated cellulosic fiber nonwoven fabric of the embodiment (or index of uniformity in texture or formation or roughness in a dry state) is 250 or lower, preferably 230 or lower and more preferably 210 or lower. If the dry formation index of the chemical impregnation nonwoven fabric is higher than 250, then the nonwoven fabric is nonuniform, resulting in more gaps with the skin, reduced ability to follow the skin and lower adhesiveness. In order to adjust the formation index to within this range it is effective to use a fabric with a fine mesh as the lower layer net laminated with the spun sheet, with a mesh number of 60 or greater being particularly preferred, and it is also effective to increase the number of nozzles and to increase the number of water streams for hydroentangling.

The initial tensile strength of the regenerated cellulosic fiber nonwoven fabric of the embodiment in the "weft direction of the nonwoven fabric" when wet is 0.25 N/50 mm to 0.75 N/50 mm, preferably 0.35 N/50 mm to 0.75 N/50 mm and more preferably 0.45 N/50 mm to 0.75 N/50 mm. If the initial tensile strength is lower than 0.25 N/50 mm, the ductility will be high and the handleability will be notably reduced when the sheet is worn on the face. If it is higher than 0.75 N/50 mm, on the other hand, elongation will be inhibited and adhesiveness will be notably reduced when the sheet is worn on the face. In order to adjust the initial tensile strength in the "weft direction of the nonwoven fabric" when wet to within the range specified above, it is effective to increase the number of spinnerets and the number of fibers, and ensure tangling strength by a high-pressure stream jet.

The phrase "weft direction of the nonwoven fabric" as used herein refers to the direction perpendicular to the pull direction in which the initial tensile strength is greatest during measurement of the initial tensile strength of the nonwoven fabric when wet.

The phrase "nonwoven fabric when wet (wetted state)" means the state where the nonwoven fabric has been dipped for 1 hour in grade 3 water at 20°C $\pm$2°C according to ISO3696.

The phrase "dry state of the nonwoven fabric", as used herein, means the state after standing for 16 hours or longer in a thermostatic chamber at 20°C, 65% RH after drying to a constant mass at 105°C.

The basis weight of the regenerated cellulosic fiber nonwoven fabric of the embodiment is preferably 10 g/m$^2$ to 80 g/m$^2$, more preferably 10 g/m$^2$ to 60 g/m$^2$ and even more preferably 20 g/m$^2$ to 50 g/m$^2$. If the basis weight is less than 10 g/m$^2$, the fabric will be too thin and the handleability will be notably reduced to an impractical level. If the basis weight is higher than 80 g/m$^2$, on the other hand, handleability will be superior but the thickness will not allow movement of the skin to be followed, and adhesiveness will be reduced.

The thickness of the regenerated cellulosic fiber nonwoven fabric of the embodiment is preferably 0.05 mm to 0.50 mm, more preferably 0.05 mm to 0.40 mm and even more preferably 0.15 mm to 0.40 mm. A thickness of smaller than 0.05 mm will be too thin, notably reducing the handleability. A thickness of larger than 0.50 mm, on the other hand, will notably reduce the adhesiveness.

The bulk density of the regenerated cellulosic fiber nonwoven fabric of the embodiment is preferably 0.15 g/cm$^3$ to 0.30 g/cm$^3$. If the bulk density is outside of this range the liquid absorption and adhesiveness will be reduced.

The moisture absorption speed of the regenerated cellulosic fiber nonwoven fabric of the embodiment is preferably 175 mm or higher. If the moisture absorption speed is lower than 175 mm the adhesiveness will be reduced.

A method for producing the regenerated cellulosic fiber nonwoven fabric of the embodiment will now be described.

In production of the nonwoven fabric, the fiber size can be varied by the spinneret used in the spinning step, and the amount of stock solution and the amount of spinning solution flowing down through it.

By varying the shape of the lower layer net laminated with the spun sheet, the perforating pressure, and the speed, it is possible to vary the surface form, thickness and nonwoven fabric strength.

In order to adjust the dry surface roughness and dry formation index to within the predetermined ranges it is effective to use a fabric comprising a fine mesh as the lower layer net laminated with the spun sheet. Using a dense lower layer net, i.e. one with a fine mesh, will result in smaller irregularities on the nonwoven fabric surface caused by water pressure during high-pressure stream jet treatment. The preferred mesh number for the lower layer net is 60 mesh or greater, with 70 mesh or greater being more preferred. When ultrafine fibers have been hydroentangled on a dense mesh, however, liquid permeability of the water stream through the net is poor, causing the water stream to scatter on the net and often resulting in disorder of the surface fibers. It is even more effective to combine this with a method of increasing the number of hydroentangling nozzles. Increasing the number of hydroentangling nozzles reduces the impact force of each water stream but also allows the number of water streams to be increased. Increasing the number of water streams can inhibit disorder of the surface fibers while ensuring tangling force between the fibers due to a greater number of entanglement points, thus allowing the strength to be maintained or increased.

In the method for producing a regenerated cellulosic fiber nonwoven fabric of the embodiment, the production conditions described above were employed to adjust the fiber size, surface roughness, formation and strength to within the predetermined ranges. This allowed excellent levels to be achieved for handleability when wet, adhesiveness and tactile property, even with a low basis weight.

EXAMPLES

The present invention will now be explained in greater detail using examples and comparative examples, with the understanding that the invention is not limited to the examples.

The measuring methods used in the Examples and Comparative Examples will be explained first. For nonwoven fabrics that had already been dipped in liquids such as cosmetic liquids, they were first rinsed once with purified water and dried at 105°C to a constant mass, after which they were allowed to stand for 16 hours or longer in a thermostatic chamber at 20°C, 65% RH, and then measured.

(1) Fiber size ($\mu$m)

The nonwoven fabric was observed using a JSM-6380 scanning electron microscope by JEOL Corp. at 10,000x magnification, 50 fibers were arbitrarily selected, and the average value of the measurements was recorded as the fiber size.

(2) Dry surface roughness ($\mu$m) (dry surface roughness Sq ($\mu$m))

The term "surface roughness" refers to the root mean square of the heights at each of the points in the measured region, and it corresponds to the standard deviation for "height". The term "height" in this case refers to the distance from the reference surface at each point on the measurement surface. The dry surface roughness of a 5 cm-square nonwoven fabric was measured using a one-shot 3D profile meter by Keyence Corp. Measurement was carried out three times and the average value was recorded as the surface roughness ($\mu$m).

(3) Dry formation index (index of uniformity in texture or formation or roughness in a dry state)

The formation index of a nonwoven fabric of size 5 cm $\times$ 5 cm or greater was measured using a formation tester (FMT-M III by Nomura Shoji Co., Ltd.).

(4) Initial tensile strength in weft direction of nonwoven fabric when wet (N/50 mm) (wet CD direction: 10% Mo.(N))

A 5 cm $\times$ 10 cm nonwoven fabric was measured using a TENSILON tensile tester by Orientech Co. at a constant rate of 300 mm/min while in the wetted state, and the strength at 10% elongation in the weft direction of the nonwoven fabric was measured. Measurement was performed 5 times and the average value was recorded as the initial tensile strength (N/50 mm). When a sample size suited for the measurement method cannot be obtained, a 5 cm $\times$ 5 cm fabric may be used instead and comparison made with conversion to the sample size.

(5) Basis weight (g/m$^2$)

A cellulose nonwoven fabric with an area of 0.05 m$^2$ or greater was dried at 105°C to a fixed mass, and then allowed to stand for 16 hours or longer in a thermostatic chamber at 20°C, 65% RH, the mass was measured, and the mass (g) per square meter of the nonwoven fabric was determined.

(6) Thickness (mm)

The dried nonwoven fabric was measured by the thickness test of JIS-L1096, with a load of 1.96 kPa.

(7) Bulk density (g/cm$^3$)

The bulk density was calculated by the following formula.

$$\text{Bulk density (g/cm}^3) = \text{Basis weight (g/m}^2) \div \text{thickness (mm)} \div 1000$$

(8) Moisture absorption speed (mm) (moisture absorption speed (mm, MD, 10 min))

The moisture absorption speed is the value obtained in a Byreck test according to JIS-L1907. Specifically, a test piece that is 250 mm long in the widthwise direction is sampled with a 25 mm width, and after dipping it in water for 10 minutes to 10 mm at the lower end, the dipped height from the water surface is measured as the moisture absorption speed.

(9) Adsorption drag (gf)

A wetted 10 cm × 10 cm nonwoven fabric that had been dipped for 1 hour in grade 3 water at 20°C ±2°C according to ISO3696 was set on a high performance artificial skin-simulating Bioskin Plate by Beaulax Co. A hook-shaped wire was passed through the center of the nonwoven fabric, while a TENSILON tensile tester by Orientech Co. was used to lift up the other end of the wire at a constant rate of 200 mm/min, measuring the load when the nonwoven fabric separated from the simulated skin. Measurement was performed 5 times and the average value was recorded as the adsorption drag (gf). A larger adsorption drag indicates superior adhesiveness.
The methods for evaluation and measurement for a face mask will now be described.

(10) Handleability

The nonwoven fabric punched into the form of a face mask was folded 4 times and impregnated with 25 cc of a commercially available cosmetic liquid (DHC Noumitsu Urumi Hada by DHC Corp.), the ease of opening the sample was assessed by each of 10 females by evaluation on the following 5-level scale, and the average value was calculated as an index of the handleability. An average evaluation of less than 3.5 points was defined as undesirable handleability.

    5 points: Can be unfolded without wrinkling.
    4 points: Can be unfolded, with wrinkling but without frustration.
    3 points: Normal.
    2 points: Can be unfolded eventually but with frustration.
    1 point: Cannot be unfolded.

(11) Adhesiveness

The nonwoven fabric punched into the form of a face mask was impregnated with 25 cc of a commercially available cosmetic liquid (DHC Noumitsu Urumi Hada by DHC Corp.), the adhesiveness on skin was assessed by each of 10 females by evaluation on the following 5-level scale, and the average value was calculated as an index of the adhesiveness. An average evaluation of less than 3.5 points was defined as undesirable adhesiveness.

    5 points: Satisfactory with no gaps between base material and skin.
    3 points: Small gaps between base material and skin.
    1 point: Gaps between base material and skin, readily detachable.

(12) Tactile property

The tactile property was organoleptically evaluated by 20 participants. The evaluation method and assessment level were as described below, and the average value for the 20 evaluators was recorded as the evaluation value for the tactile property of the sample.
Evaluation method: The inside of the left upper arm was gently rubbed with a 10 cm × 10 cm wet sample, and assessment was made by evaluation on the following 5-level scale. An average evaluation of less than 3.5 points was defined as an undesirable tactile property.

    5: Soft, with no discernible stimulation on the skin.
    4: Smooth stimulating feel.
    3: Slightly rough feel.
    2: Rough stimulating feel.
    1: Prickly stimulating feel.

[Example 1]

Cotton linter was dissolved in a copper ammonia solution and a spinneret having $180.9/cm^2$ solution discharge holes with diameters of 0.3 mm was used for continuous spinning under flow tension with a throughput of 0.034 cc/min per hole in four layers onto a net to form a sheet, tangling the fibers by a 3 MPa high-pressure stream jet while forming through-holes and recesses in the sheet, which was then dried. The hole-opening conditions were a 70 mesh lower layer net and $3.70$ hole/$cm^2$ hydroentangling nozzles. The lower layer net speed was 37.5 m/min. The fiber size of the obtained nonwoven fabric was 5.25 $\mu$m, the dry surface roughness was 84 $\mu$m, the dry formation index was 173 and the initial tensile strength in the weft direction of the nonwoven fabric when wet was 0.26 N/50 mm.

[Example 2]

A nonwoven fabric was obtained by the same method as Example 1, except that the lower layer net speed was changed so that the basis weight of the nonwoven fabric was 10.4 $g/m^2$. The fiber size of the obtained nonwoven fabric was 4.51 $\mu$m, the dry surface roughness was 89 $\mu$m, the dry formation index was 184 and the initial tensile strength in the weft direction of the nonwoven fabric when wet was 0.30 N/50 mm.

[Example 3]

A nonwoven fabric was obtained by the same method as Example 1, except that the lower layer net speed was changed so that the basis weight of the nonwoven fabric was 38.1 $g/m^2$. The fiber size of the obtained nonwoven fabric was 6.52 $\mu$m, the dry surface roughness was 51 $\mu$m, the dry formation index was 152 and the initial tensile strength in the weft direction of the nonwoven fabric when wet was 0.48 N/50 mm.

[Example 4]

A nonwoven fabric was obtained by the same method as Example 1, except that the lower layer net speed was changed so that the basis weight of the nonwoven fabric was 47.9 $g/m^2$. The fiber size of the obtained nonwoven fabric was 5.43 $\mu$m, the dry surface roughness was 44 $\mu$m, the dry formation index was 140 and the initial tensile strength in the weft direction of the nonwoven fabric when wet was 0.39 N/50 mm.

[Example 5]

A nonwoven fabric was obtained by the same method as Example 1, except that the lower layer net speed was changed so that the basis weight of the nonwoven fabric was 59.8 $g/m^2$. The fiber size of the obtained nonwoven fabric was 6.45 $\mu$m, the dry surface roughness was 39 $\mu$m, the dry formation index was 131 and the initial tensile strength in the weft direction of the nonwoven fabric when wet was 0.43 N/50 mm.

[Example 6]

A nonwoven fabric was obtained by the same method as Example 1, except that the lower layer net speed was changed so that the basis weight of the nonwoven fabric was 79.7 $g/m^2$. The fiber size of the obtained nonwoven fabric was 7.01 $\mu$m, the dry surface roughness was 30 $\mu$m, the dry formation index was 119 and the initial tensile strength in the weft direction of the nonwoven fabric when wet was 0.55 N/50 mm.

[Example 7]

A nonwoven fabric was obtained by the same method as Example 3, except that the spinneret diameter was changed so that the fiber size of the nonwoven fabric was 9.92 $\mu$m. The dry surface roughness of the obtained nonwoven fabric was 81 $\mu$m, the dry formation index was 150 and the initial tensile strength in the weft direction of the nonwoven fabric when wet was 0.28 N/50 mm.

[Example 8]

A nonwoven fabric was obtained by the same method as Example 1, except that the spinneret diameter was changed so that the fiber size of the nonwoven fabric was 1.21 $\mu$m. The dry surface roughness of the obtained nonwoven fabric was 52 $\mu$m, the dry formation index was 177 and the initial tensile strength in the weft direction of the nonwoven fabric when wet was 0.36 N/50 mm.

[Example 9]

A nonwoven fabric was obtained by the same method as Example 4, except that the mesh number of the lower layer net was 65 mesh and the speed was increased. The fiber size of the obtained nonwoven fabric was 4.72 $\mu$m, the dry surface roughness was 97 $\mu$m, the dry formation index was 211 and the initial tensile strength in the weft direction of the nonwoven fabric when wet was 0.35 N/50 mm.

[Example 10]

A nonwoven fabric was obtained by the same method as Example 4, except that the mesh number of the lower layer net was 65 mesh and the speed was decreased. The fiber size of the obtained nonwoven fabric was 3.27 $\mu$m, the dry surface roughness was 77 $\mu$m, the dry formation index was 244 and the initial tensile strength in the weft direction of the nonwoven fabric when wet was 0.40 N/50 mm.

[Example 11]

A nonwoven fabric was obtained by the same method as Example 1, except that the lower layer net speed was decreased and the perforating pressure was changed to 3.5 MPa. The fiber size of the obtained nonwoven fabric was 7.31 $\mu$m, the dry surface roughness was 71 $\mu$m, the dry formation index was 197 and the initial tensile strength in the weft direction of the nonwoven fabric when wet was 0.73 N/50 mm.

[Example 12]

A nonwoven fabric was obtained by the same method as Example 1, except that the lower layer net speed was increased and the perforating pressure was changed to 2.5 MPa. The fiber size of the obtained nonwoven fabric was 6.06 $\mu$m, the dry surface roughness was 49 $\mu$m, the dry formation index was 130 and the initial tensile strength in the weft direction of the nonwoven fabric when wet was 0.26 N/50 mm.

[Example 13]

A 55.4 g/m$^2$ card web was formed using a spunlace production apparatus, with 0.8 dtex Bemberg™ staple fibers by Asahi Kasei Corp. (material: cupro) × 51 mm cotton. This was followed by treatment with a web support mesh woven fabric using a 70 mesh aperture, 3.70 hole/cm$^2$ hydroentangling nozzles, and water stream pressure of 1 MPa on the front and 2 MPa on the back, and then treatment again with 3 MPa from the front side and drying. This series of steps was carried out at a speed of 40 m/min. A heated roll processor was then used for smoothing to obtain a nonwoven fabric. The fiber size of the obtained nonwoven fabric was 8.29 $\mu$m, the dry surface roughness was 65 $\mu$m, the dry formation index was 161 and the initial tensile strength in the weft direction of the nonwoven fabric when wet was 0.68 N/50 mm.

[Example 14]

A nonwoven fabric was obtained by the same method as Example 13, except that the spinning stock solution was changed to staple fibers prepared from a solidifying solution of cotton linter with N-methylmorpholine N-oxide as the solvent. The fiber size of the obtained nonwoven fabric was 8.14 $\mu$m, the dry surface roughness was 62 $\mu$m, the dry formation index was 152 and the initial tensile strength in the weft direction of the nonwoven fabric when wet was 0.60 N/50 mm.

[Example 15]

A nonwoven fabric was obtained by the same method as Example 1, except that the spinning stock solution was changed to a solidifying solution of cotton linter with N-methylmorpholine N-oxide as the solvent. The fiber size of the obtained nonwoven fabric was 5.16 $\mu$m, the dry surface roughness was 87 $\mu$m, the dry formation index was 208 and the initial tensile strength in the weft direction of the nonwoven fabric when wet was 0.30 N/50 mm.

[Example 16]

A nonwoven fabric was obtained by the same method as Example 8, except that an ammonia water solution was used to prepare a 2-fold diluted spinning stock solution. The fiber size of the obtained nonwoven fabric was 0.52

μm, the dry surface roughness was 64 μm, the dry formation index was 185 and the initial tensile strength in the weft direction of the nonwoven fabric when wet was 0.25 N/50 mm. In Example 16, the spinning stock solution was diluted two-fold with an ammonia water solution, the cellulose concentration was 1/2, and the fiber size, basis weight and thickness were all approximately 1/2, compared to Example 8. The monofilament strength was therefore lower and the wet strength was reduced. The monofilament was lighter, scattering of the surface fibers increased to some extent and the dry surface roughness and formation were impaired.

[Comparative Example 1]

A nonwoven fabric was obtained under the conditions described in Japanese Unexamined Patent Publication No. 2009-297535, Example 1, except that the throughput per hole was 0.040 cc/min, the lower layer net speed was 29.7 m/min and the lower layer net was specially folded. The number of hydroentangling nozzles was 2.88 hole/cm$^2$. The fiber size of the obtained nonwoven fabric was 5.97 μm, the dry surface roughness was 128 μm, the dry formation index was 293 and the initial tensile strength in the weft direction of the nonwoven fabric when wet was 0.35 N/50 mm. Compared to Example 3, the surface roughness and formation index were greater, and although the handleability when wet was not particularly problematic, the adhesiveness and tactile property were impaired.

[Comparative Example 2]

A nonwoven fabric was obtained under the conditions described in Japanese Unexamined Patent Publication No. 2005-120519, Example 1, except that the throughput per hole was 0.037 cc/min, the lower layer net speed was 34.1 m/min and the lower layer net was 20 mesh with four layers. The number of hydroentangling nozzles was 3.70 hole/cm$^2$. The fiber size of the obtained nonwoven fabric was 6.29 μm, the dry surface roughness was 129 μm, the dry formation index was 308 and the initial tensile strength in the weft direction of the nonwoven fabric when wet was 0.18 N/50 mm. Compared to Example 3, the surface roughness and formation index were greater, the initial tensile strength was low, and the handleability when wet, the adhesiveness and the tactile property were all impaired.

[Comparative Example 3]

Cotton linter was dissolved in a copper ammonia solution and a spinneret having 45/cm$^2$ solution discharge holes with diameters of 0.6 mm was used for continuous spinning under flow tension with a throughput of 0.09 cc/min per hole in six layers onto a net to form a sheet, tangling the fibers by a high-pressure stream jet while forming through-holes and recesses in the sheet, which was then dried. The hole-opening conditions were a 40 mesh lower layer net and 3.70 hole/cm$^2$ hydroentangling nozzles. The lower layer net speed was 45.5 m/min. The fiber size of the obtained nonwoven fabric was 11.8 μm, the dry surface roughness was 143 μm, the dry formation index was 263 and the initial tensile strength in the weft direction of the nonwoven fabric when wet was 0.39 N/50 mm. Compared to Example 4, the fiber size was large, the surface roughness and formation index were greater, and the handleability when wet, the adhesiveness and the tactile property were all impaired.

[Comparative Example 4]

A nonwoven fabric was obtained by the same method as Comparative Example 3, except that the lower layer net was changed to 70 mesh. The fiber size of the obtained nonwoven fabric was 12.1 μm, the dry surface roughness was 98 μm, the dry formation index was 192 and the initial tensile strength in the weft direction of the nonwoven fabric when wet was 0.28 N/50 mm. Compared to Example 6, the fiber size was large, and the handleability when wet, the adhesiveness and the tactile property were all reduced.

[Comparative Example 5]

A commercially available regenerated cellulose fiber nonwoven fabric (lyocell nonwoven fabric, Sontara (trade name) by Jacob Holm Co.) was measured for its properties and evaluated. The fiber size of the obtained nonwoven fabric was 11.1 μm, the dry surface roughness was 85 μm, the dry formation index was 294 and the initial tensile strength in the weft direction of the nonwoven fabric when wet was 1.03 N/50 mm. Compared to Example 13, the fiber size was larger, the formation index was greater, and although the handleability when wet was not particularly problematic, the adhesiveness and tactile property were impaired.

[Comparative Example 6]

A nonwoven fabric was obtained under the conditions described in Japanese Unexamined Patent Publication No. 2018-127744, Example 1, except that the stock solution throughput and lower layer net speed were changed so that the fiber size of the nonwoven fabric was 5.45 $\mu$m and the basis weight of the nonwoven fabric was 60 g/m$^2$. The hole-opening conditions were a 70 mesh lower layer net and 2.88 hole/cm$^2$ hydroentangling nozzles. The fiber size of the obtained nonwoven fabric was 5.45 $\mu$m, the dry surface roughness was 110 $\mu$m, the dry formation index was 221 and the initial tensile strength in the weft direction of the nonwoven fabric when wet was 0.32 N/50 mm. Compared to Example 8, the surface roughness was higher, more gaps were formed with the skin, and the adhesiveness and tactile property were notably reduced.

[Comparative Example 7]

A nonwoven fabric was obtained under the conditions described in Japanese Unexamined Patent Publication No. 2015-70968, Example 1, except that the stock solution throughput, lower layer net speed and perforating pressure were changed. The hole-opening conditions were a 70 mesh lower layer net and 2.88 hole/cm$^2$ hydroentangling nozzles. The fiber size of the obtained nonwoven fabric was 6.81 $\mu$m, the dry surface roughness was 94 $\mu$m, the dry formation index was 219 and the initial tensile strength in the weft direction of the nonwoven fabric when wet was 0.23 N/50 mm. Compared to Example 11, the initial tensile strength in the weft direction of the nonwoven fabric when wet was lower, the nonwoven fabric was more easily elongated, and the handleability when wet was notably reduced.

[Comparative Example 8]

A nonwoven fabric was obtained by the same method as Example 1, except that the lower layer net was 40 mesh, and the stock solution throughput and lower layer net speed were changed. The fiber size of the obtained nonwoven fabric was 3.35 $\mu$m, the dry surface roughness was 80 $\mu$m, the dry formation index was 260 and the initial tensile strength in the weft direction of the nonwoven fabric when wet was 0.38 N/50 mm. Compared to Example 9, the formation index was larger, the chemical solution migration was poorer and the adhesiveness was notably reduced.

[Comparative Example 9]

A nonwoven fabric was obtained under the conditions described in Japanese Unexamined Patent Publication No. 2018-127744, Example 3. The hole-opening conditions were a 40 mesh lower layer net and 2.88 hole/cm$^2$ hydroentangling nozzles. The fiber size of the obtained nonwoven fabric was 7.3 $\mu$m, the dry surface roughness was 120 $\mu$m, the dry formation index was 278 and the initial tensile strength in the weft direction of the nonwoven fabric when wet was 0.25 N/50 mm. Compared to Example 14, the surface roughness and formation index were greater, and although the handleability when wet was not particularly problematic, the adhesiveness and tactile property were impaired.

The properties of the nonwoven fabrics obtained in Examples 1 to 16 and Comparative Examples 1 to 9 are shown in Table 1-1 and Table 1-2, respectively.

[Table 1]

Table 1-1

| | Fiber size (μm) | Dry surface roughness Sq (μm) | Formation index | Wet CD direction 10% Mo. (N) | Basis weight (g/m²) | Thickness (mm) | Adsorption drag (gf) | Bulk density (g/cm³) | Moisture absorption speed (mm, MD, 10 min) | Organoleptic evaluation (handleability) | Organoleptic evaluation (adhesiveness) |
|---|---|---|---|---|---|---|---|---|---|---|---|
| Example 1 | 5.25 | 84 | 173 | 0.26 | 25.3 | 0.16 | 47.7 | 0.16 | 177 | 4.0 | 4.1 |
| Example 2 | 4.51 | 89 | 184 | 0.30 | 10.4 | 0.06 | 46.6 | 0.17 | 175 | 4.2 | 4.0 |
| Example 3 | 6.52 | 51 | 152 | 0.48 | 38.1 | 0.24 | 55.9 | 0.16 | 184 | 4.8 | 4.8 |
| Example 4 | 5.43 | 44 | 140 | 0.39 | 47.9 | 0.30 | 54.7 | 0.16 | 202 | 4.5 | 4.7 |
| Example 5 | 6.45 | 39 | 131 | 0.43 | 59.8 | 0.37 | 50.0 | 0.16 | 194 | 4.6 | 4.3 |
| Example 6 | 7.01 | 30 | 119 | 0.55 | 79.7 | 0.50 | 48.9 | 0.16 | 176 | 4.6 | 4.2 |
| Example 7 | 9.92 | 81 | 150 | 0.28 | 38.4 | 0.25 | 48.9 | 0.15 | 180 | 3.8 | 4.2 |
| Example 8 | 1.21 | 52 | 177 | 0.36 | 25.6 | 0.14 | 52.4 | 0.18 | 210 | 4.4 | 4.5 |
| Example 9 | 4.72 | 97 | 211 | 0.35 | 45.1 | 0.25 | 46.6 | 0.18 | 185 | 4.2 | 4.0 |
| Example 10 | 3.27 | 77 | 244 | 0.40 | 50.8 | 0.32 | 45.4 | 0.16 | 183 | 4.2 | 3.9 |
| Example 11 | 7.31 | 71 | 197 | 0.73 | 38.3 | 0.24 | 43.1 | 0.16 | 180 | 4.9 | 3.7 |
| Example 12 | 6.06 | 49 | 130 | 0.26 | 15.3 | 0.10 | 52.4 | 0.15 | 196 | 3.6 | 4.5 |
| Example 13 | 8.29 | 65 | 161 | 0.68 | 55.4 | 0.26 | 44.2 | 0.21 | 201 | 4.7 | 3.8 |

(continued)

| | Fiber size (μm) | Dry surface roughness Sq (μm) | Formation index | Wet CD direction 10% Mo. (N) | Basis weight (g/m²) | Thickness (mm) | Adsorption drag (gf) | Bulk density (g/cm³) | Moisture absorption speed (mm, MD, 10 min) | Organoleptic evaluation (handleability) | Organoleptic evaluation (adhesiveness) |
|---|---|---|---|---|---|---|---|---|---|---|---|
| Example 14 | 8.14 | 62 | 152 | 0.60 | 40.1 | 0.24 | 45.4 | 0.17 | 188 | 4.6 | 3.9 |
| Example 15 | 5.16 | 87 | 208 | 0.30 | 35.1 | 0.23 | 44.2 | 0.15 | 190 | 4.4 | 3.8 |
| Example 16 | 0.52 | 64 | 185 | 0.25 | 12.7 | 0.07 | 50.0 | 0.18 | 244 | 3.8 | 4.3 |

[Table 2]

Table 1-2

| | Fiber size (μm) | Dry surface roughness Sq (μm) | Formation index | Wet CD direction 10% Mo. (N) | Basis weight (g/m²) | Thickness (mm) | Adsorption drag (gf) | Bulk density (g/cm³) | Moisture absorption speed (mm, MD, 10 min) | Organoleptic evaluation (handleability) | Organoleptic evaluation (adhesiveness) |
|---|---|---|---|---|---|---|---|---|---|---|---|
| Comp. Example 1 | 5.97 | 128 | 293 | 0.35 | 39.5 | 0.36 | 26.8 | 0.11 | 173 | 3.8 | 2.3 |
| Comp. Example 2 | 6.29 | 129 | 308 | 0.18 | 30.7 | 0.30 | 27.9 | 0.10 | 149 | 2.7 | 2.4 |
| Comp. Example 3 | 11.8 | 143 | 263 | 0.39 | 38.1 | 0.31 | 24.4 | 0.12 | 157 | 3.4 | 2.1 |
| Comp. Example 4 | 12.1 | 98 | 192 | 0.28 | 38.8 | 0.30 | 40.7 | 0.13 | 144 | 3.2 | 3.5 |
| Comp. Example 5 | 11.1 | 85 | 294 | 1.03 | 36.9 | 0.28 | 26.8 | 0.13 | 127 | 4.2 | 2.3 |
| Comp. Example 6 | 5.45 | 110 | 221 | 0.32 | 59.2 | 0.26 | 37.2 | 0.23 | 120 | 3.9 | 3.2 |
| Comp. Example 7 | 6.81 | 94 | 219 | 0.23 | 28.4 | 0.22 | 39.6 | 0.13 | 111 | 2.1 | 3.4 |
| Comp. Example 8 | 3.35 | 80 | 260 | 0.38 | 50.1 | 0.38 | 36.1 | 0.13 | 136 | 3.6 | 3.1 |
| Comp. Example 9 | 7.30 | 120 | 278 | 0.25 | 38.0 | 0.18 | 32.6 | 0.21 | 125 | 3.7 | 2.8 |

INDUSTRIAL APPLICABILITY

The regenerated cellulosic fiber nonwoven fabric of the invention is a nonwoven fabric material that, even with a low basis weight, has excellent handleability when wet, high adhesiveness and an excellent tactile property, and it can therefore be suitably used as a base material for a chemical impregnation sheet such as a face mask, point sheet or three-dimensional mask.

**Claims**

1. A nonwoven fabric composed of regenerated cellulosic fibers with a fiber size, determined as described in the description, of 0.5 $\mu$m to 10.0 $\mu$m, wherein the nonwoven fabric has a dry surface roughness, determined as described in the description, of 100 $\mu$m or lower, a dry formation index, determined as described in the description, of 250 or less, and an initial tensile strength in the weft direction of the nonwoven fabric when wet, determined as described in the description, of 0.25 N/50 mm to 0.75 N/50 mm, whereby the "weft direction of the nonwoven fabric" refers to the direction perpendicular to the pull direction in which the initial tensile strength is greatest during measurement of the initial tensile strength of the nonwoven fabric when wet.

2. The nonwoven fabric according to claim 1, wherein the basis weight of the nonwoven fabric is 10 g/m$^2$ to 80 g/m$^2$, and the thickness is 0.05 mm to 0.50 mm.

3. The nonwoven fabric according to claim 1 or 2, wherein the cellulosic fibers are continuous long fibers.

4. The nonwoven fabric according to any one of claims 1 to 3, wherein the bulk density is 0.15 g/cm$^3$ to 0.30 g/cm$^3$.

5. Use of the nonwoven fabric according to any one of claims 1 to 4 as a chemical impregnation sheet.

6. Use according to claim 5, wherein the chemical impregnation sheet is a face mask.

**Patentansprüche**

1. Vliesstoff, zusammengesetzt aus regenerierten Cellulosefasern mit einer Fasergröße, die so bestimmt wird, wie es in der Beschreibung beschrieben ist, von 0,5 $\mu$m bis 10,0 $\mu$m, wobei der Vliesstoff eine Trockenoberflächenrauigkeit, die so bestimmt wird, wie es in der Beschreibung beschrieben ist, von 100 $\mu$m oder weniger, einen Trockenformationsindex, der so bestimmt wird, wie es in der Beschreibung beschrieben ist, von 250 oder weniger und eine Anfangszugfestigkeit in Schussrichtung des Vliesstoffs im feuchten Zustand, die so bestimmt wird, wie es in der Beschreibung beschrieben ist, von 0,25 N/50 mm bis 0,75 N/50 mm aufweist, wobei sich "Schussrichtung des Vliesstoffs" auf die Richtung senkrecht zur Zugrichtung bezieht, in der die Anfangszugfestigkeit während der Messung der Anfangszugfestigkeit des Vliesstoffs im feuchten Zustand am größten ist.

2. Vliesstoff gemäß Anspruch 1, wobei das Flächengewicht des Vliesstoffs 10 g/m$^2$ bis 80 g/m$^2$ beträgt und die Dicke 0,05 mm bis 0,50 mm beträgt.

3. Vliesstoff gemäß Anspruch 1 oder 2, wobei die Cellulosefasern lange Endlosfasern sind.

4. Vliesstoff gemäß einem der Ansprüche 1 bis 3, wobei die Rohdichte 0,15 g/cm$^3$ bis 0,30 g/cm$^3$ beträgt.

5. Verwendung des Vliesstoffs gemäß einem der Ansprüche 1 bis 4 als mit Chemikalien imprägniertes Flächengebilde.

6. Verwendung gemäß Anspruch 5, wobei das mit Chemikalien imprägnierte Flächengebilde eine Gesichtsmaske ist.

**Revendications**

1. Textile non tissé constitué de fibres cellulosiques régénérées avec une taille de fibre, déterminée comme décrit dans la description, de 0,5 $\mu$m à 10,0 $\mu$m, dans lequel le textile non tissé présente une rugosité de surface sèche, déterminée comme décrit dans la description, de 100 $\mu$m ou inférieure, un indice de formation à sec, déterminé comme décrit dans la description, de 250 ou inférieur, et une résistance à la traction initiale dans la direction de trame du textile non tissé lorsque humide, déterminée comme décrit dans la description, de 0,25 N/50 mm à 0,75 N/50 mm, sur quoi la "direction de trame du textile non tissé" fait référence à la direction perpendiculaire à la direction de tirage dans laquelle la résistance à la traction initiale est la plus importante pendant la mesure de la résistance à la traction initiale du textile non tissé lorsque humide.

2. Textile non tissé selon la revendication 1, dans lequel la masse de base du textile non tissé est de 10 g/m$^2$ à 80 g/m$^2$, et l'épaisseur est de 0,05 mm à 0,50 mm.

3. Textile non tissé selon la revendication 1 ou 2, dans lequel les fibres cellulosiques sont des fibres longues continues.

4. Textile non tissé selon l'une quelconque des revendications 1 à 3, dans lequel la densité apparente est de 0,15 g/cm$^3$ à 0,30 g/cm$^3$.

5. Utilisation du textile non tissé selon l'une quelconque des revendications 1 à 4 comme une feuille d'imprégnation chimique.

6. Utilisation selon la revendication 5, dans laquelle la feuille d'imprégnation chimique est un masque de visage.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4816312 B
- JP 6385191 B
- JP 6267913 B
- JP 2018127744 A

- JP 2009297535 A
- JP 2005120519 A
- JP 2015070968 A